# EUROPEAN PATENT SPECIFICATION

(11) **EP 2 588 054 B1**
(45) Date of publication and mention of the grant of the patent: **23.12.2020**
(21) Application number: 11730518.5
(22) Date of filing: 28.06.2011
(51) Int. Cl.: A61F 13/15

(54) **ABSORBENT ARTICLE HAVING AN IMPROVED LEG CUFF**
ABSORBIERENDER ARTIKEL MIT VERBESSERTEM ABSCHLUSS AN DEN BEINTEILEN
ARTICLE ABSORBANT AVEC AMELIORATION DU BOURRELET ENTOURANT LA JAMBE

(30) Priority: 29.06.2010 US 359457 P
(43) Date of publication of application: 08.05.2013
(73) Proprietor: The Procter & Gamble Company, Cincinnati, OH 45202 (US)
(72) Inventor: LASH, Glen, Ray, Cincinnati Ohio 45239 (US)
(74) Representative: P&G Patent Germany
(86) International application number: PCT/US2011/042173
(87) International publication number: WO 2012/003184

(56) References cited:
- FR-A1- 2 699 813
- US-A- 5 624 426

## Description

### FIELD OF THE INVENTION

The present invention relates to absorbent articles such as incontinence pads, and more particularly, the present invention relates to absorbent articles having leg cuffs with a generally quadrilateral cross-sectional geometry.

### BACKGROUND OF THE INVENTION

Side leakage is a common problem for absorbent articles used for urinary incontinence. Gushes of urine discharge can temporarily flood the surface of the absorbent article, leaking over the side margin. To address this problem, others have incorporated elastic gathering elements in various ways along the lateral edge of the absorbent article.

Some articles have gathered elastic members along the side margins. The elastic members are placed sufficiently away from the relatively stiff absorbent core to allow contraction during wear. The gathered edges can create edge barriers to resist urine leakage at the sides. However, with these designs, the side elastic members are in physically close proximity to the elastic of the underwear of the user. The elastics of the underwear frequently dominate the elastics of the absorbent article, rendering them ineffective at preventing side leakage.

Other articles utilize gathered elastic members to create standing barriers along the side surface edges of the article. In these designs, the elastics are incorporated into material that is attached to the body-side surface layer of the absorbent article. During wear, the intent is for the elastics to lift from the surface of the article, creating standing barriers on each side of the article, preventing spread of urine. However, these designs are not reliable for preventing side leakage because bunching of the absorbent material can cause material of the side barriers to be pushed into the fluid path, preventing the absorbent core from working effectively. Further, many of these designs are uncomfortable to wear because the elastics create a harsh edge which rubs against the wearer. US5624426 discloses a disposable absorbent article including leg cuffs having a proximal edge, a distal edge and an elasticized region disposed there between. The elasticized region has an inner edge, an outer edge and a width. Each leg cuff includes an inner bond disposed adjacent at least a portion of the proximal edge of each leg cuff. An outer bond is spaced laterally outwardly from the inner bond, the spacing between the inner bond and said outer bond defining a leg cuff base width. Each leg cuff also includes an inner wall that extends upwardly and laterally outwardly from the inner bond to the inner edge of the elasticized region, and an outer wall that extends upwardly and laterally inwardly from the outer bond to the outer edge of the elasticized region. An elastic element is disposed in the elasticized region of each leg cuff. FR2699SI3A relates to a pad, e.g. for a baby's diaper or adult incontinence pad, comprising lengthwise air circulation channels on each side of the absorbent layer. Each channel contains two lengthwise elastic elements, located on the inside of fold lines spaced some 15-25 mm apart, and the inner surface of each channel's outside wall is lined with a strip of a moisture-resistant and gas permeable material.

As such, there remains a need for an absorbent article for urinary incontinence having improved side leakage protection.

### SUMMARY OF THE INVENTION

According to the present invention, such object is achieved by an absorbent article having the characteristics of claim 1. Further, preferred embodiments are covered by the dependent claims.

### BRIEF DESCRIPTION OF THE DRAWINGS

While the specification concludes with claims particularly pointing out and distinctly claiming the present invention, it is believed that the present invention will be better understood from the following description in conjunction with the accompanying drawings.
Figure 1 is a top plan view of an absorbent article that is an incontinence pad with portions cut-away to more clearly show the construction of the incontinence pad;
Figure 2 is a cross-sectional view of the absorbent article of Figure 1 taken along section line 2-2 after contraction of the elastics;
Figure 3 is a cross-sectional view of the absorbent article of Figure 1 taken along section line 2-2 prior to contraction of the elastics.
Figure 4 is a cross-sectional view of the absorbent article of Figure 1 taken along section line 4-4 after contraction of the elastics.

### DETAILED DESCRIPTION OF THE INVENTION

The present invention relates to absorbent articles having improved leg cuffs. The leg cuffs have a generally quadrilaterial cross-sectional geometry, such as, for example, a trapezoid. Generally, when the absorbent article is applied to underwear by the user, elastic contraction within the leg cuff causes the leg cuff to lift and provide an improved absorbent article having raised side edges. In use, the leg cuffs are pressed up against the user's skin around the perineal area by the user's undergarment thereby forming a gasketing effect. Bodily fluids can then be directed toward the middle of the absorbent article to keep urine within the absorbent area of the absorbent article. Surprisingly, it has been discovered that the quadrilateral leg cuff configuration provides improved comfort and side leakage protection. In addition, the quadrilateral leg cuff configuration prevents the leg cuff from being distorted and bunched towards the longtitudinal centerline of the absorbent article, so that it does not risk blocking the fluid path to the absorbent material.

As used herein, the term "absorbent article" refers to devices which absorb and contain body exudates, and, more specifically, refers to devices which are placed against or in proximity to the body of the wearer to absorb and contain the various exudates discharged from the body. Absorbent articles include incontinence pads, sanitary napkins, disposable diapers, incontinence briefs, training pants and the like.

The term "disposable" is used herein to describe absorbent articles which are not intended to be laundered or otherwise restored or reused as an absorbent article (i.e., they are intended to be discarded after a single use, and, preferably, to be recycled, composted or otherwise disposed of in an environmentally compatible manner).

The term "longitudinal" as used herein, refers to a line, axis or direction in the plane of the absorbent article that is generally aligned with (e.g., approximately parallel to) a vertical plane which bisects a standing wearer into left and right body halves when the absorbent article is worn.

As used herein the term "permanently joined" refers to a connection that cannot be unattached without at least partially destroying one of the attached components.

As used herein, the term "quadrilateral" refers to a shape having four sides and four corners. In certain embodiments, the corners can be straight corners. Alternatively, in certain embodiments, one or more of the corners can be rounded corners. In addition, in certain embodiments, the sides of the quadrilateral can be flexible.

As used herein the term "releasably joined" refers to a connection that is meant to be easily released.

The terms "transverse" or "lateral" as used herein, are interchangeable, and refer to a line, axis or direction which lies within the plane of the absorbent article that is generally perpendicular to the longitudinal direction.

As used herein, the term "trapezoid" refers to a shape having four sides and four corners, with at least one pair of parallel sides.

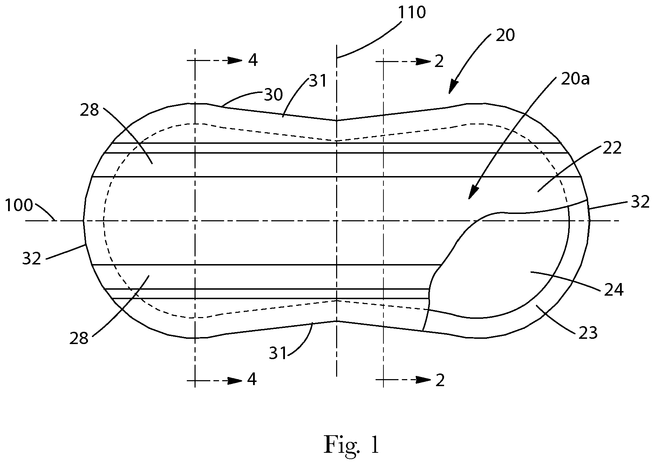

Figure 1 is a plan view of an absorbent article 20 of the present invention with the portion of the absorbent article 20 which faces or contacts the wearer oriented towards the viewer. As shown in Figure 1, the absorbent article 20 includes a liquid pervious topsheet 22, a liquid impervious backsheet 23 joined with the topsheet 22, and an absorbent core 24 positioned between the topsheet 22 and the backsheet 23. Two leg cuffs 28 are secured to the absorbent article 20. The leg cuffs 28 are disposed one at each side of the absorbent article 20 in a spaced relation to each other.

The absorbent article 20 has two surfaces, a body-contacting surface or body facing surface 20a and a garment facing surface 20b. The absorbent article 20 is shown in Figure 1 as viewed from its body facing surface 20a. The body facing surface 20a is intended to be worn adjacent to the body of the wearer while the garment facing surface 20b is on the opposite side and is intended to be placed adjacent to the wearer's undergarments when the absorbent article 20 is worn. The absorbent article 20 has two centerlines, a longitudinal centerline 100 and a transverse centerline 110. Figure 1 also shows that the absorbent article 20 has a periphery 30 which is defined by the outer edges of the absorbent article 20 in which the longitudinal edges (or "side edges") are designated 31 and the end edges (or "ends") are designated 32.

Figure 1 shows one embodiment of the absorbent article 20 in which the topsheet 22 and the backsheet 23 have length and width dimensions generally larger than those of the absorbent core 24. The topsheet 22 and the backsheet 23 extend beyond the edges of the absorbent core 24 to thereby form portions of the periphery 30.

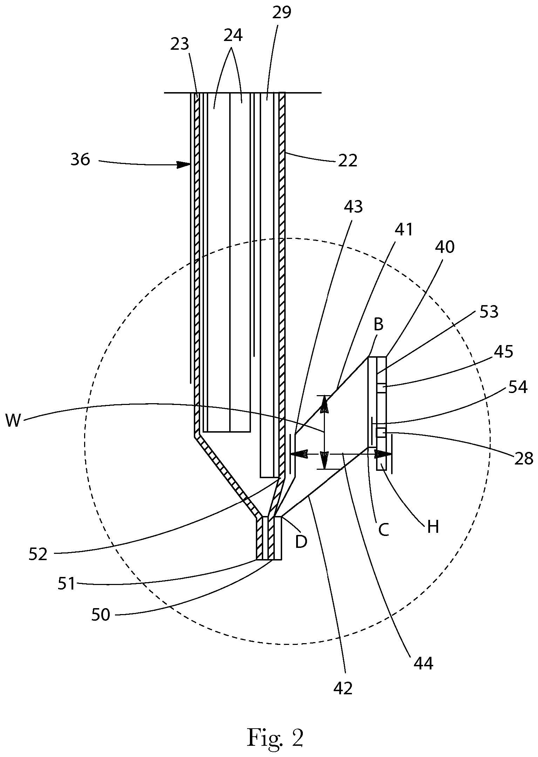

Figure 2 is a partial cross-sectional view of the absorbent article 20 taken along section line 2--2 of Figure 1 when leg cuff 28 is in the lifted position, such as, for example, after contraction of elastics 45. In certain embodiments, as shown in Figure 2, the absorbent article 20 can include an absorbent core 24 and/or a secondary topsheet 29. The absorbent core 24 can be in any suitable configuration, such as, for example, in one or more layers, c-folded, or any other suitable configuration. As can be seen in Figure 2, the absorbent article 20 can include an adhesive fastener 36 for attaching the absorbent article 20 to the undergarment of the wearer. In certain embodiments, a removable release liner can cover the adhesive fastener 36 to keep the adhesive from sticking to a surface other than a crotch portion of the undergarment prior to use.

As shown in Figure 2, leg cuffs 28 have a quadrilateral cross-section in the lifted position. The quadrilateral cross-section is formed by upper surface 40, an inner barrier 41, an outer barrier 42 opposite the inner barrier 41, and a lower surface 43 opposite the upper surface 40. Elastic 45 is provided on all or a portion of upper surface 40.

As shown in Figure 2, when the absorbent article 20 is applied to underwear by the user, the elastic 45 contraction within the leg cuff 28 causes the leg cuff 28 to lift. In the lifted position, leg cuffs 28 have a quadrilateral cross-section formed by upper surface 40, inner barrier 41, outer barrier 42, and lower surface 43. The quadrilateral cross-section is formed by the upper surface 40, inner barrier 41, outer barrier 42, and lower surface 43 connecting at points A, B, C, and D, where A is a point of transition between the inner barrier 41 and lower surface 43, such as, e.g., at the point of leg cuff 28 attachment to the topsheet 22; B is a point of transition between leg cuff 28 and the upper surface 40; C is a point of transition between upper surface 40 and outer barrier 42; and D is a point of transition between leg cuff 28 and lower surface 43.

The upper surface 40 and the lower surface 43 are opposite and separate from one another when the leg cuffs 28 are in the lifted position, and inner barrier 41 and outer barrier 42 are opposite and separate from one another when the leg cuffs 28 are in the lifted position. Elastic 45 can be provided along all or a portion of upper surface 40 such that elastic 45 is generally parallel to lower surface 43 when the leg cuffs 28 are in the lifted position.

As shown in Figure 2, the leg cuffs 28 have a height H, a width W, and a volume 44. Volume 44 is considered the open area formed when the leg cuff is in the lifted position. Generally, the total volume can be determined using the length of the open area when the leg cuff 28 is lifted, such as, for example, the length of the leg cuff 28 where upper surface 40 is not bonded to the topsheet 22 or any other suitable portion of the absorbent article 20.

Inner barrier 41 serves to keep urine within the absorbent area of the absorbent article. Upper surface 40 is the elastic containing surface of the leg cuff. This surface is free to fit flat against the body, and to spreads the elastic force over a distance suitable for good wearing comfort. Outer barrier 42 provides a second barrier for protection in the event urine wicks through the inner barrier. In addition, outer barrier 42 keeps the leg cuff from being distorted and bunched towards the longtitudinal centerline of the absorbent article, so that it does not risk blocking the fluid path to the absorbent material. In certain embodiments, lower surface 43 facilitates lifting of inner barrier 41 and outer barrier 42, such as, for example, by preventing underlying construction adhesives from contacting the surfaces.

As shown in Figure 2, leg cuffs 28 can be joined to the absorbent article 20 in any suitable manner, such as, for example, using adhesive 50 and/or perimeter crimp seal 51. Lower surface 43 is attached to topsheet 22 using adhesive 52. The elastic 45 containing portion of the leg cuff 28 is folded back onto leg cuff 28 to form an improved body contacting surface, namely a triple layer body contacting surface 53. The elastic 45 containing portion can be folded and joined to leg cuff 28 in any suitable manner, such as, for example, using adhesive 54.

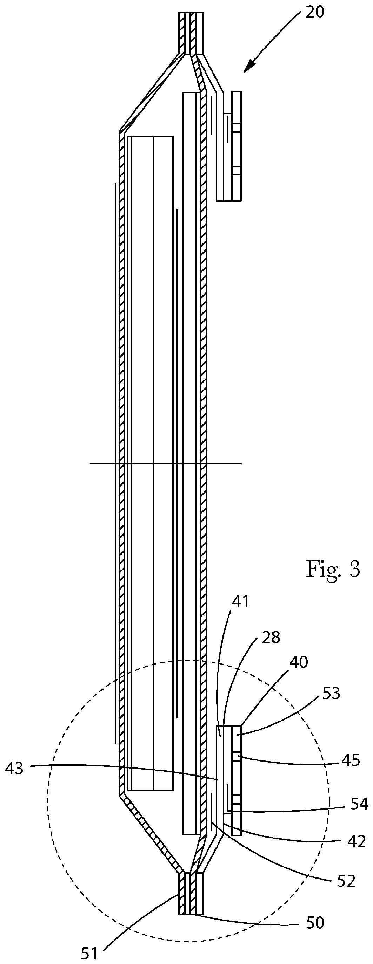

As shown in Figure 3, in certain embodiments, leg cuffs 28 include an upper surface 40, an inner barrier 41, an outer barrier 42 opposite the inner barrier 41, and a lower surface 43 opposite the upper surface 40; however, when the leg cuff 28 is in the flat position, the upper surface 40, the lower surface 43, inner barrier 41, and outer barrier 42 are flattened together. As shown in Figure 3, leg cuffs 28 can be joined to the absorbent article 20 in any suitable manner, such as, for example, using adhesive 50 and/or perimeter crimp seal 51. Lower surface 43 is attached to topsheet 22 using adhesive 52. In addition, the elastic 45 containing portion of the leg cuff 28 is folded back onto leg cuff 28 to form an improved body contacting surface, namely a triple layer body contacting surface 53. The elastic 45 containing portion can be folded and joined to leg cuff 28 in any suitable manner, such as, for example, using adhesive 54.

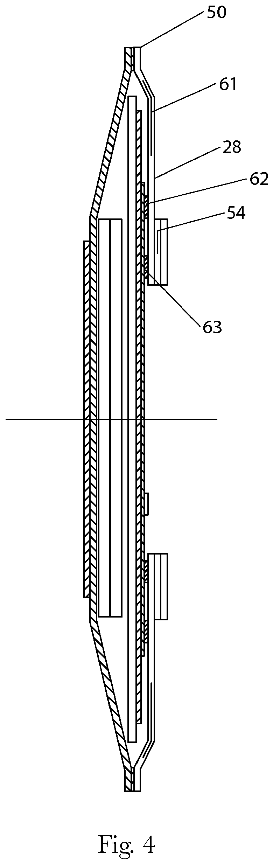

As shown in Figure 4, in certain embodiments, leg cuffs 28 can be bonded along the absorbent article 20 length using adhesive 61, 62, and or 63, or other suitable bonding agents, such as, for example by folding the leg cuff 28 back on itself and bonding the folded portion to a portion of the absorbent article 20. For example, in certain embodiments, the folded back portion can be bonded at the ends of the absorbent article to the topsheet, such as, for example, using adhesive or other suitable bonding agents. This configuration prevents the leg cuff 28 from lifting at the ends of the absorbent article while allowing it to lift in the central portion of the absorbent article.

To construct the leg cuffs, the elastic strands can be put under tension by stretching them. In certain embodiments, the elastic can be stretched to about 200% to about 400%, such as, for example, from about 250% to about 350% of its original relaxed dimension. In one example, the elastic can have a length of x and can be stretched an additional 2.5x such that the final stretched length of the elastic is 3.5x. In another example, the elastic can have a length of x and can be stretched an additional 2x such that the final stretched length of the elastic is 3x. In yet another example, the elastic can have a length of x and can be stretched an additional 1.5x such that the final stretched length is 2.5x. The elastics are then attached to the nonwoven, such as, for example, by gluing using elastic wrap adhesive or other suitable adhesives. In certain embodiments, the glued length of the elastic can be any suitable length, such as, for example, from about 100 to about 500 mm, from about 100 to about 400 mm, from about 100 to about 300 mm, from about 100 to about 200 mm, from about 150 to about 200 mm, or any other suitable length, centered along the length of the absorbent article. When the elastics are cut at the ends of the absorbent article, they attempt to contract to their relaxed dimension.

The leg cuff nonwoven can be bonded to the topsheet of the absorbent article, such as, for example, by a slot coated stripe of adhesive, glue beads, ultrasonic sealing, or other suitable bonding agents. In certain embodiments, the leg cuff nonwoven can be bonded to the backsheet at the peripheral margins of the absorbent article, such as, for example, using a crimp or other suitable bonding agents, such as, for example, adhesive.

The portion of the leg cuff having the elastic members is then folded back on itself and the folded back portion can be glued continuously along the absorbent article length. The folded back portion can then be bonded intermittently at the ends of the absorbent article to the topsheet to prevent the leg cuff from lifting at the ends of the absorbent article while allowing it to lift in the central portion of the absorbent article.

The absorbent core may be any absorbent that is capable of absorbing or retaining liquids (e.g., menses and/or urine). The absorbent core has a body facing surface, a garment facing surface, longitudinal edges, and end edges. The absorbent core may be manufactured from a wide variety of liquid-absorbent materials commonly used in absorbent articles and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these. The configuration and construction of the absorbent core may also be varied (e.g., the absorbent core may have varying caliper zones (e.g., profiled so as to be thicker in the center), hydrophilic gradients, superabsorbent gradients, or lower density and lower average basis weight acquisition zones; or may comprise one or more layers or structures). The total absorbent capacity of the absorbent core should, however, be compatible with the design loading and the intended use of the absorbent article. Further, the size and absorbent capacity of the absorbent core may be varied to accommodate different uses.

The absorbent article can have an absorbent core to absorb and store bodily fluids discharged during use. In some embodiments of incontinence pads, pantiliners, sanitary napkins, or other such devices of the present invention, an absorbent core is not necessary, the pad consisting only of a topsheet (that can have some absorbency) and a fluid impermeable backsheet. Absorbent core can be formed from any suitable materials, such as, for example, multiple plies of creped cellulose wadding, fluffed cellulose fibers, wood pulp fibers also known as airfelt, textile fibers, a blend of fibers, a mass or batt of fibers, airlaid webs of fibers, a web of polymeric fibers, and a blend of polymeric fibers.

In certain embodiments, the absorbent core can be relatively thin, such as, for example, less than about 10 mm, or less than about 5 mm in thickness, or less than about 3 mm, or less than about 1 mm in thickness. Thickness can be measured by any means known in the art for doing so while the core is under a uniform pressure of 1.7 kPa (0.25 psi).

In certain embodiments, the absorbent core can comprise absorbent gelling materials (AGM), including AGM fibers, as is known in the art.

The absorbent core can be formed or cut to a shape, the outer edges of which define a core periphery. The shape of absorbent core can be generally rectangular, circular, oval, elliptical, or the like. In certain embodiments, the absorbent core can be folded, such as, for example, c-folded. Alternatively, the absorbent core can be one or more layers or stacks, such as, for example, a one ply core, a two ply core, a three ply core, and the like. In addition, in certain embodiments, the absorbent core can be generally centered with respect to the longitudinal centerline L and transverse centerline T.

To prevent absorbed bodily exudates from contacting the wearer's garments, in certain embodiments, the absorbent article can have a liquid impermeable backsheet. The backsheet can comprise any materials suitable for backsheets, such as, for example, polymer films and film/nonwoven laminates. To provide a degree of softness and vapor permeability for the garment-facing side of the absorbent article, in certain embodiments, backsheet can be a vapor permeable outer layer on the garment-facing side of the absorbent article. In certain embodiments, the backsheet can be formed from any suitable vapor permeable material, such as, for example, a microporous film, an apertured formed film, or other polymer film that is vapor permeable, or rendered to be vapor permeable. One suitable material is a soft, smooth, compliant, vapor pervious material, such as a nonwoven web that is hydrophobic or rendered hydrophobic to be substantially liquid impermeable. A nonwoven web provides for softness and conformability for comfort, and can be low noise producing so that movement does not cause unwanted sound.

To provide for softness next to the body, in certain embodiments, the absorbent article can have a body-facing layer, referred to herein as topsheet. The topsheet can be formed from any soft, smooth, compliant, porous material which is comfortable against human skin and through which fluids such as urine or vaginal discharges can pass, such as, for example, fibrous nonwoven webs and can comprise any suitable fibers, such as, for example, including bicomponent and/or shaped fibers. In certain embodiments, the topsheet can also be a liquid permeable polymer film, such as an apertured film, or an apertured formed film as is known on sanitary napkins such as ALWAYS® brand sanitary napkins.

At least one of the topsheet and backsheet can define a shape, the edge of which defines an outer periphery of the absorbent article. In certain embodiments, both the topsheet and backsheet define the absorbent article outer periphery. The two layers can be die cut, for example, after combining all the components into the structure of the absorbent article as described herein. However, in certain embodiments, the shape of either the topsheet or backsheet can be independently defined.

In certain embodiments, at least one fluid permeable secondary topsheet can be interposed between the absorbent core and topsheet. The secondary topsheet can aid in rapid acquisition and/or distribution of fluid and can be in fluid communication with the absorbent core. In certain embodiments, the secondary topsheet does not completely cover the absorbent core, but it can extend laterally to the core periphery. Alternatively, the topsheet, secondary topsheet, or the absorbent core can be layered structures, the layers facilitating fluid transport by differences in fluid transport properties, such as capillary pressure.

In certain embodiments, the absorbent core does not extend laterally outward to the same extent as either the topsheet or backsheet, but the absorbent article outer periphery can be substantially larger than the core outer periphery. In this manner, the region of the absorbent article between the core periphery and the absorbent article outer periphery can define a breathable zone that permits vapors to go through portions of the sanitary napkin, thereby escaping and providing for dryer comfort when worn. The breathable zone incontinence pad having a breathable zone can be according to the teachings of U.S. Ser. No. 10/790,418, filed March 1, 2004.

All the components can be adhered together with adhesives, including hot melt adhesives, as is known in the art. The adhesive can be Findlay H2128 UN or Savare PM 17 and can be applied using a Dynafiber HTW system.

In use, the absorbent article can be held in place by any support or attachment suitable for such purposes. In certain embodiments, the absorbent article is placed in the user's undergarment or panty and secured thereto by a fastener such as an adhesive. The adhesive secures the absorbent article in the crotch portion of the user's panty. Thus, a portion or all of the outer surface of the backsheet is coated with adhesive. Any adhesive or glue suitable for such purposes can be used for the adhesive herein, such as, for example, using pressure-sensitive adhesive. Suitable adhesives include, for example, Century A-305-IV manufactured by the Century Adhesives Corporation of Columbus, Ohio; and Instant Lock 34-2823 manufactured by the National Starch and Chemical Company of Bridgewater, N.J. Suitable adhesive fasteners are also described in U.S. Pat. No. 4,917,697. Before the absorbent article is placed in use, the pressure-sensitive adhesive is typically covered with a removable release liner in order to keep the adhesive from drying out or adhering to a surface other than the crotch portion of the panty prior to use. Suitable release liners are also described in U.S. Pat. Nos. 4,917,697 and 4,556,146. Any commercially available release liners commonly used for such purposes can be utilized herein. Non-limiting examples of suitable release liners are BL30MG-A Silox E1/0 and BL30MG-A Silox 4P/0 both of which are manufactured by the Akrosil Corporation of Menasha, Wis. The absorbent article can be used by removing the release liner and thereafter placing the absorbent article in a panty so that the adhesive contacts the panty. The adhesive maintains the absorbent article in its position within the panty during use. The release liner can also be a wrapper that can individually package the absorbent article.

The absorbent article can include a colored portion that can be one or more colors, including for example, black, red, blue, violet, orange, yellow, green, and indigo as well as any declination thereof or mixture thereof. The colored portion can be provided in any suitable location on the absorbent article. In one example, the colored portion is visible from the body-facing surface of the absorbent article, and is provided on the body-facing surface and/or the garment-facing surface of the topsheet, on an insert positioned between the topsheet and the absorbent core, and/or as a part of the absorbent core whereby the colored portion is viewable from the viewing surface of the topsheet. Suitable colored portions are described in, for example, U.S. Pat. No. 7,241,280. In addition, all or a portion of the leg cuffs can be colored. For example, the leg cuffs can be colored so that they are distinguishable from a region adjacent the leg cuffs. In addition, or alternatively, the leg cuffs can be colored to coordinate with another region of the absorbent article, such as, for example, a colored portion viewable from a central region of the body-facing surface of the absorbent article.

A plurality of absorbent articles can be disposed in a package. Suitable packages include, for example, bags, boxes, and the like in any suitable shape and formed from any suitable material. In addition, the absorbent articles can be individually packaged in wrappers. The wrappers can include color and/or design indicators that can be used to signal absorbency or other article performance characteristics such as size and strength. In one example, the package can have a window, such as, for example, as described in U.S. Pat. No. 7,185,761, and the absorbent articles can be visible through the window.

## Claims

1. An absorbent article (20) comprising a topsheet (22), a backsheet (23) joined to the topsheet (22); and an absorbent core (24) positioned between the topsheet (22) and the backsheet (23), the absorbent core (24) having longitudinal edges and end edges;
the absorbent article (20) having a leg cuff (28) extending along each longitudinal edge of the absorbent core, the leg cuff (28) having a lifted position and a flat position, the leg cuff having a height (H), a width (W), a length, a volume (44), an upper surface (40), an inner barrier (41), an outer barrier (42), a lower surface (43), and a cross-sectional area that is a generally quadrilateral shape in the lifted position, the cross-sectional area being defined by the upper surface (40), the inner barrier (41), the outer barrier (42), and the lower surface (43), wherein the lower surface (43) is attached to the topsheet (22) using adhesive (52), and wherein the upper surface (40) comprises an elastic material (45),
**characterized in that** the elastic material (45) containing portion of the leg cuff (28) is folded back onto leg cuff (28) to form a triple layer body contacting surface (53).

2. The absorbent article (20) of claim 1, wherein at least a portion of the upper surface (40) and the lower surface (43) are opposite and separate from one another when the leg cuffs (28) are in the lifted position, and at least a portion of the inner barrier (41) and the outer barrier (42) are opposite and separate from one another when the leg cuffs (28) are in the lifted position.

3. The absorbent article (20) of any of claims 1 or 2, wherein at least a portion of the upper surface (40) and the lower surface (43) are generally parallel to one another when the leg cuffs (28) are in the lifted position.

4. The absorbent article (20) of claim 1 or 2, wherein none of the upper surface (40), the inner barrier (41), the outer barrier (42), and the lower surface (43) are parallel to one another when the leg cuffs (28) are in the lifted position.

5. The absorbent article (20) of any of claims 1 to 4, wherein one or more of the upper surface (40), the inner barrier (41), the outer barrier (42), and the lower surface (43) comprise nonwoven material.

6. The absorbent article (20) of any of claims 1 to 5, wherein the absorbent article is an incontinence pad.

7. The absorbent article (20) of any of claims 1 to 6, wherein the upper surface (40) is permanently joined at the ends of the absorbent article to the absorbent article such that the leg cuffs (28) are prevented from moving to the lifted position at the end portions.

8. The absorbent article (20) of any of claims 1 to 7, wherein the lifted position is provided in the central portion.

9. The absorbent article (20) of any of claims 1 to 8, wherein the elastic material (45) is stretched more than 200% of its relaxed dimension prior to attachment to the absorbent article.

## Patentansprüche

1. Absorptionsartikel (20), umfassend eine Oberschicht (22), eine Unterschicht (23), die mit der Oberschicht (22) verbunden ist; und einen Absorptionskern (24), der zwischen der Oberschicht (22) und der Unterschicht (23) angeordnet ist, wobei der Absorptionskern (24) Längsränder und Endränder aufweist;
wobei der Absorptionsartikel (20) ein Beinbündchen (28) aufweist, das sich entlang jedes Längsrands des Absorptionskerns erstreckt, wobei das Beinbündchen (28) eine angehobene Position und eine flache Position aufweist, wobei das Beinbündchen eine Höhe (H), eine Breite (W), eine Länge, ein Volumen (44), eine obere Oberfläche (40), eine innere Barriere (41), eine äußere Barriere (42), eine untere Oberfläche (43) und eine Querschnittsfläche mit einer in der angehobenen Position im Allgemeinen vierseitigen Form aufweist, wobei die Querschnittsfläche durch die obere Oberfläche (40), die innere Barriere (41), die äußere Barriere (42) und die untere Oberfläche (43) definiert ist, wobei die untere Oberfläche (43) unter Verwendung von Klebstoff (52) an der Oberschicht (22) befestigt ist und wobei die obere Oberfläche (40) ein elastisches Material (45) umfasst,
**dadurch gekennzeichnet, dass** das elastische Material (45), das einen Abschnitt des Beinbündchens (28) enthält, auf das Beinbündchen (28) zurückgefaltet ist, um eine dreischichtige Körperkontaktfläche (53) zu bilden.

2. Absorptionsartikel (20) nach Anspruch 1, wobei mindestens ein Abschnitt der oberen Oberfläche (40) und der unteren Oberfläche (43) einander gegenüberliegen und voneinander getrennt sind, wenn sich die Beinbündchen (28) in der angehobenen Position befinden, und mindestens ein Abschnitt der inneren Barriere (41) und der äußeren Barriere (42) einander gegenüberliegen und voneinander getrennt sind, wenn sich die Beinbündchen (28) in der angehobenen Position befinden.

3. Absorptionsartikel (20) nach einem der Ansprüche 1 oder 2, wobei mindestens ein Abschnitt der oberen Oberfläche (40) und der unteren Oberfläche (43) im Allgemeinen parallel zueinander sind, wenn sich die Beinbündchen (28) in der angehobenen Position befinden.

4. Absorptionsartikel (20) nach Anspruch 1 oder 2, wobei keine der oberen Oberfläche (40), der inneren Barriere (41), der äußeren Barriere (42) und der unteren Oberfläche (43) parallel zueinander sind, wenn sich die Beinbündchen (28) in der angehobenen Position befinden.

5. Absorptionsartikel (20) nach einem der Ansprüche 1 bis 4, wobei eine oder mehrere der oberen Oberfläche (40), der inneren Barriere (41), der äußeren Barriere (42) und der unteren Oberfläche (43) Vliesmaterial umfassen.

6. Absorptionsartikel (20) nach einem der Ansprüche, 1 bis 5, wobei der Absorptionsartikel eine Inkontinenzeinlage ist.

7. Absorptionsartikel (20) nach einem der Ansprüche 1 bis 6, wobei die obere Oberfläche (40) an den Enden des Absorptionsartikels dauerhaft mit dem Absorptionsartikel verbunden ist, sodass die Beinbündchen (28) daran gehindert werden, sich an den Endabschnitten in die angehobene Position zu bewegen.

8. Absorptionsartikel (20) nach einem der Ansprüche 1 bis 7, wobei die angehobene Position im zentralen Abschnitt bereitgestellt ist.

9. Absorptionsartikel (20) nach einem der Ansprüche 1 bis 8, wobei das elastische Material (45) vor dem Befestigen an dem Absorptionsartikel um mehr als 200 % seiner entspannten Abmessung gedehnt wird.

## Revendications

1. Article absorbant (20) comprenant une feuille de dessus (22), une feuille de fond (23) jointe à la feuille de dessus (22) ; et une âme absorbante (24) positionnée entre la feuille de dessus (22) et la feuille de fond (23), l'âme absorbante (24) ayant des bords longitudinaux et des bords extrêmes ;
l'article absorbant (20) ayant un revers de jambe (28) s'étendant le long de chaque bord longitudinal de l'âme absorbante, le revers de jambe (28) ayant une position relevée et une position plate, le revers de jambe ayant une hauteur (H), une largeur (W), une longueur, un volume (44), une surface supérieure (40), une barrière interne (41), une barrière externe (42), une surface inférieure (43), et une aire en coupe transversale qui est une forme généralement quadrilatérale dans la position relevée, l'aire en coupe transversale étant définie par la surface supérieure (40), la barrière interne (41), la barrière externe (42), et la surface inférieure (43), dans lequel la surface inférieure (43) est fixée à la feuille de dessus (22) en utilisant un adhésif (52), et dans lequel la surface supérieure (40) comprend un matériau élastique (45),
**caractérisé en ce que** le matériau élastique (45) contenant une partie du revers de jambe (28) est replié sur le revers de jambe (28) pour former une surface en contact avec le corps à triple couche (53).

2. Article absorbant (20) selon la revendication 1, dans lequel au moins une partie de la surface supérieure (40) et la surface inférieure (43) sont opposées et séparées l'une par rapport à l'autre lorsque les revers de jambe (28) sont dans la position relevée, et au moins une partie de la barrière interne (41) et la barrière externe (42) sont opposées et séparées l'une par rapport à l'autre lorsque les revers de jambe (28) sont dans la position relevée.

3. Article absorbant (20) selon l'une quelconque des revendications 1 ou 2, dans lequel au moins une partie de la surface supérieure (40) et la surface inférieure (43) sont généralement parallèles l'une à l'autre lorsque les revers de jambe (28) sont dans la position relevée.

4. Article absorbant (20) selon la revendication 1 ou 2, dans lequel aucune parmi la surface supérieure (40), la barrière interne (41), la barrière externe (42) et la surface inférieure (43) n'est parallèle à l'autre lorsque les revers de jambe (28) sont dans la position relevée.

5. Article absorbant (20) selon l'une quelconque des revendications 1 à 4, dans lequel une ou plusieurs parmi la surface supérieure (40), la barrière interne (41), la barrière externe (42) et la surface inférieure (43) comprennent un matériau non tissé.

6. Article absorbant (20) selon l'une quelconque des revendications 1 à 5, dans lequel l'article absorbant est une serviette d'incontinence.

7. Article absorbant (20) selon l'une quelconque des revendications 1 à 6, dans lequel la surface supérieure (40) est jointe de manière permanente au niveau des extrémités de l'article absorbant à l'article absorbant de telle sorte que les revers de jambe (28) sont empêchés de passer à la position relevée au niveau des parties d'extrémité.

8. Article absorbant (20) selon l'une quelconque des revendications 1 à 7, dans lequel la position relevée est fournie dans la partie centrale.

9. Article absorbant (20) selon l'une quelconque des revendications 1 à 8, dans lequel le matériau élastique (45) est étiré de plus de 200 % de sa dimension relâchée avant fixation à l'article absorbant.
